# EUROPEAN PATENT APPLICATION

(11) **EP 3 581 391 A1**
(43) Date of publication of application: **18.12.2019**
(21) Application number: 19179534.3
(22) Date of filing: 11.06.2019
(51) Int. Cl.: B41J 3/407

(54) **MARKINGS ON MARIJUANA AND METHOD THEREFOR**

(30) Priority: 11.06.2018 US 201862683175 P
(71) Applicant: Ceccarelli, Loreto J., Vaughan, Ontario L4K 0B2 (CA)
(72) Inventor: Ceccarelli, Loreto J., Vaughan, Ontario L4K 0B2 (CA)
(74) Representative: Jones, David Alan

(57) **Abstract**

In a method for applying distinguishing markings to a marijuana bud, one or more indicia are directly applied to an irregular outer surface of the marijuana bud. The indicia may be applied to the marijuana bud by spraying, stamping or dipping the marijuana bud with a liquid marking composition including a liquid base and a colored substance. In the method, the bud may be moved along a conveyor with a plurality of other buds, and the liquid marking composition is sprayed directly onto the irregular outer surface of the marijuana bud from spray nozzles located above the conveyor. An apparatus for applying indicia to a plurality of marijuana buds includes a conveyor having a plurality of laterally-extending dividers spaced apart along the moving direction. The dividers define a plurality of channels, each having a width sufficient to closely receive a marijuana bud.

## Description

### Cross-reference to Related Application

This application claims the benefit of and priority to United States Provisional Patent Application No. 62/683,175, filed on June 11, 2018, entitled "METHOD OF MARIJUANA MARKING," which is hereby expressly incorporated by reference.

### Technical Field

This disclosure relates to methods for direct labelling of plant material derived from the marijuana plant.

### Background

The legalization of marijuana for medical and/or recreational use brings with it some unique branding challenges. A large number of marijuana varieties are currently produced by a multitude of producers and each variety has unique characteristics. For example, they may contain different chemicals, or the amount of the same chemical may differ among the varieties. The different varieties may have different effects on the user, and they may require different methods of processing and/or consumption for best results.

Although legalized marijuana products are frequently sold in labelled packaging, the marijuana buds are sold without labelling to clearly identify the source or other information of the product to the end consumer. To preserve their quality, marijuana dispensaries typically store buds in bulk containers such as glass jars or vacuum containers. While these bulk containers may contain buds of a certain variety or quality, they may lack any labelling to identify the source or the variety of the buds, let alone the particular effects, composition, and method of use for the particular variety. Furthermore, once a bud is removed from the bulk container and purchased by the end consumer, the information of the bud is not easily accessible. This causes difficulties in distinguishing marijuana products produced by different producers, to distinguish legally produced marijuana products from black market products, or to distinguish marijuana of different varieties. In addition, it makes it difficult for the distributors/retailers to handle the marijuana in the most suitable manner for the particular marijuana variety, for the user to consume marijuana in a manner best suited for the particular marijuana variety, and for processors to extract particular chemicals, such as THC (tetrahydrocannabinol) and Cannabidiol (CBD), in the most efficient way regarding the particular marijuana variety.

There exists a need for reliable labelling of marijuana products to allow consumers to distinguish between marijuana products on the basis of variety, strength, source, etc.; to allow producers to distinguish their products from their competitors' products; and to allow consumers, producers and governments to distinguish legally produced marijuana products from black market products, and to enable appropriate use of the marijuana.

### Summary

In one aspect, there is provided a method for applying distinguishing markings to a marijuana bud, comprising: applying one or more indicia directly to an irregular outer surface of the marijuana bud. In some embodiments, the method takes into account of the characteristics of the marijuana bud, for example, the shape and color, such that the indicia catches the attention of the user.

In yet another aspect, there is provided one or more indicia on a marijuana bud. The indicia and the marijuana bud cooperate such that the marijuana bud acts both as a marijuana bud and as a substrate for indicia. The indicia may contain information concerning the marijuana bud, such as the particular variety, the source of the marijuana bud, the best method to extract particular compounds from the marijuana bud of the particular variety, and the best way(s) to consume the marijuana bud. These two functions of the marijuana bud are combined to provide added product safety, reliability, and user satisfaction, etc., which would not be available absent the combination provided by the marijuana bud and the indicia. As described in more detail hereinafter, the combination provided in the invention and resulting functional interaction between the marijuana bud and the information included in the indicia that is applied on the marijuana bud makes it possible to overcome the issues in retailing, handling, processing, and consumption of marijuana bud.

According to an aspect, the method comprises applying one or more indicia directly to an irregular outer surface of the marijuana bud.

According to an aspect, the one or more indicia are applied to the marijuana bud by spraying.

According to an aspect, the one or more indicia are applied to the marijuana bud by stamping.

According to an aspect, the one or more indicia are applied to the marijuana bud by dipping.

According to an aspect, the one or more indicia are applied by spraying the marijuana bud with a liquid marking composition comprising a liquid base and a colored substance.

According to an aspect, each of the marijuana buds is moved along a conveyor with a plurality of other buds.

According to an aspect, a plurality of spray nozzles is provided above the conveyor.

According to an aspect, a liquid marking composition is sprayed directly onto the irregular outer surface of the marijuana bud from at least one of the plurality of spray nozzles.

According to an aspect, at least one divider is disposed on the conveyor to form at least one channel such that movement of the marijuana bud is restricted in at least one of a direction of conveyor movement, and a lateral direction which is transverse to the direction of conveyor movement.

According to an aspect, the liquid base is aqueous or oil-based. According to an aspect, the liquid base comprises hemp oil.

According to an aspect, the liquid marking composition further comprises one or more active ingredients derived from the marijuana plant. According to an aspect, the active ingredients are selected from one or more of THC (tetrahydrocannabinol) and CBD (cannabidiol).

According to an aspect, the colored substance comprises one or more food colorings. According to an aspect, the colored substance is selected based on the color of the marijuana bud.

According to an aspect, the indicia is stamped on the marijuana bud by at least one stamping device.

According to an aspect, the at least one stamping device comprises a flexible stamping surface.

According to an aspect, a plurality of said stamping devices are used and the stamps cooperate such that movement of the marijuana bud is restricted by the stamps.

According to an aspect, a processor is configured to control the stamping devices to cooperate.

According to an aspect, the indicia provide information regarding one or more differentiating features of the marijuana bud. According to an aspect, the one or more differentiating features of the buds are selected from one or more of source, variety and strength.

According to an aspect, the one or more differentiating features of the buds are selected from one or more of a producer's name and/or logo, a design, and color coding.

According to an aspect, the indicia further comprises instructions on using the marijuana bud.

According to an aspect, the indicia is invisible when applied. According to an aspect, the indicia is visible when heated to a predetermined temperature, under UV light, or upon chemical reaction with another material.

According to an aspect, there is provided a marijuana bud having one or more indicia applied to its outer surface.

According to an aspect, there is provided an apparatus for applying indicia to a plurality of marijuana buds.

According to an aspect, the apparatus comprises a conveyor defining a moving direction and a lateral direction which is transverse to the moving direction.

According to an aspect, the apparatus comprises a plurality of laterally-extending dividers spaced apart along the moving direction, wherein the dividers define a plurality of channels, each of the channels having a width sufficient to closely receive a marijuana bud, wherein the width of each of the channels is defined along the moving direction.

According to an aspect, the channels have different widths, such that two or more marijuana buds of similar size may be received in the same channel.

According to an aspect, the apparatus further comprises one or more plurality of spray nozzles adapted to spray the indicia onto the outer surfaces of the marijuana buds received in the channels.

According to an aspect, the nozzles are movable along one or both of the moving direction and the lateral direction.

According to an aspect, the apparatus further comprises one or more stamping devices adapted to stamp the indicia onto the outer surfaces of the marijuana buds received in the channels.

According to an aspect, each of the stamping devices comprises a flexible stamping surface.

### Brief Description of the Drawings

It will be noted that throughout the appended drawings, like features are identified by like reference numerals.

The drawings show examples of marijuana buds with applied markings.
FIG. 1 shows a marijuana bud with multiple logos.
FIG. 2 shows a marijuana bud with a single logo.
FIG. 3 shows the top view of a conveyor belt with dividers forming channels.
FIG. 4 shows a side view of the conveyor belt of FIG. 3.
FIG. 5 shows the application of indicia to a marijuana bud by stamping.

### Detailed Description

The buds of the marijuana plant, which appear after the plant enters the flowering stage of its life cycle, have a higher content of THC than other parts of the plant, and are therefore commercially prized. CBD is another commercially prized chemical from the marijuana bud.

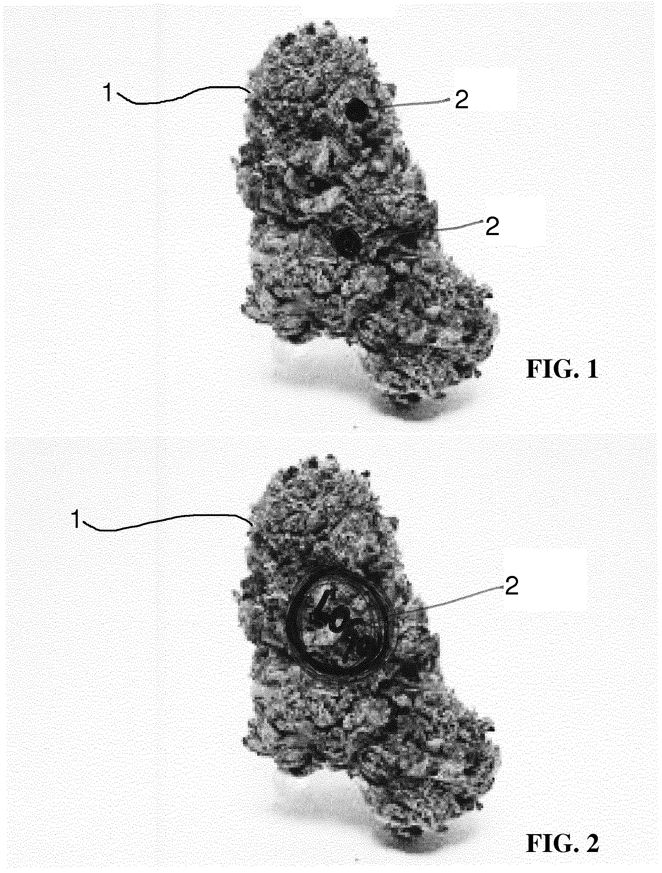

As shown in Figures 1 and 2, marijuana buds 1 are irregularly shaped and have irregular surfaces. The whole bud 1 is typically sold to the end consumer in this form, and the consumer then breaks down the bud for consumption.

The present disclosure provides a method for directly labelling marijuana buds 1 with one or more indicia 2 to identify one or more differentiating features of the buds, including source, variety, strength, etc.

According to the method, the one or more indicia are applied directly to the irregular outer surface of the marijuana bud, for example by spraying, stamping, dipping, etc.

The indicia can have any appearance and may partially or completely cover the outer surface of the bud. For example, the indicia may comprise the producer's name and/or logo, a design, color coding, the amount of the primary chemicals, for example, THC and CBD, in the particular variety of marijuana, the preferred method(s) for extracting such chemicals, and the preferred method(s) for consumption.

For example, where the indicia 2 are applied to the buds 1 by spraying, the buds 1 may be placed on an automated conveyor and passed under a plurality of spray nozzles. Due to their irregular shape, the buds 1 may become bunched together and/or tumble as they move along the conveyor, and therefore it is useful to provide a plurality of spray nozzles to ensure that most or all of the buds 1 on the conveyor will be sprayed by at least one of the nozzles. Although some of the marked buds 1 produced by the method may include only a single indicia 2, while others may have more than one indicia 2, the information conveyed by the indicia 2 will be clear to the end consumer.

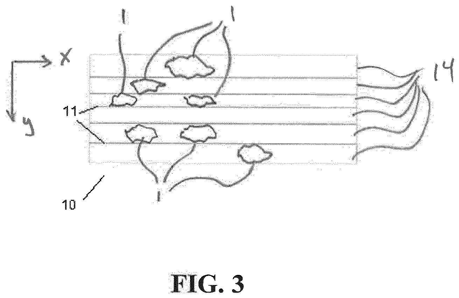

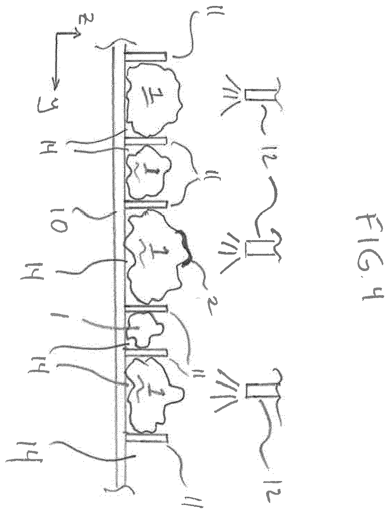

In some embodiments, as shown in Figures 3 and 4, the conveyor may be provided with mechanism to restrict the movement of the marijuana bud. For example, dividers 11 may be provided on the conveyor belt 10 to form narrow channels 14 such that the marijuana buds cannot move freely along the moving direction (y direction) and/or laterally to the moving direction of the conveyor 10 (x direction). In some embodiments, the marijuana buds 1 may be closely received in the channels 14 such that the movement along the moving (y) direction and the lateral (x) direction of the conveyor 10 is limited. In some embodiments, the channels 14 have different width to accommodate marijuana buds 1 of different sizes. For example, the marijuana buds 1 may be pre-sorted by size, and two or more buds 1 of substantially the same size may be fed into the same channel 14 such that the lateral movement can be more effectively restricted than having buds 1 of different sizes in the same channel 14.

The spraying is made easier when the movement of the marijuana buds in either direction is limited. A plurality of nozzles 12 may be provided above conveyor 10 to spray the buds 1 in each channel 14, to ensure each marijuana bud 1 in each channel 14 is sprayed. Although only three nozzles 12 are shown in Figure 4, it will be appreciated that one or more nozzles 12 may be provided for each channel 14, and/or the nozzles 12 may be movable along the x axis and/or the y axis.

In some embodiments, the plurality of nozzles 12 cooperate with each other such that multiple indicia 2, comprising the same or different information, may be sprayed on the marijuana buds 1 simultaneously or sequentially.

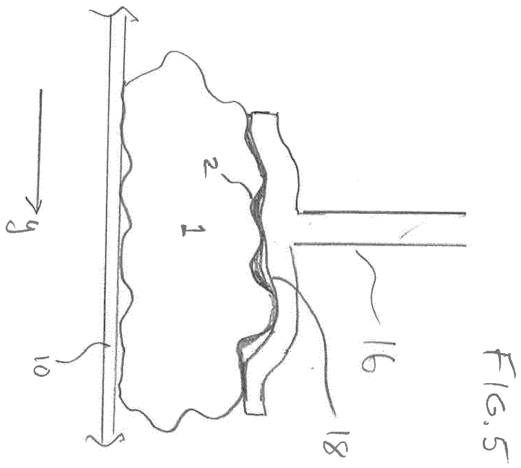

In some embodiments, as shown in Figure 5, the indicia may be applied to the buds by stamping, optionally while moving the bud 1 along a conveyor 10 in the y direction. For example, a stamping device 16 may be provided with a flexible stamping surface 18 which conforms to the surface of the marijuana bud 1 that the stamping surface is in contact with the surface of the bud 1. In some embodiments, multiple stamps 16 may be used simultaneously, contacting different surfaces of the marijuana bud 1. The multiple stamps 16 may cooperate such that the movement of the marijuana bud 1 is restricted when the indicia 2 is applied. To this end, a processor and multiple sensors may be provided to coordinate the stamping by the multiple stamps 16, including, for example, the timing, positioning, and the force exerted, etc., during stamping.

In some embodiments, when the indicia are applied by dipping, the marijuana bud may dipped in a solution such that indication color may be applied to a part or the entirety of the marijuana bud depending on the depth that the marijuana is dipped into the solution. In some embodiments, a hydrographic film, for example, a polyvinyl alcohol film, that is printed with the desired indicia is placed at the surface of liquid in a dipping device, for example, a dipping tank. The marijuana bud is then placed on the film. When the marijuana bud is pushed into the dipping device, the film wraps around the marijuana bud such that the indicia is transferred from the film to the marijuana bud. The film may then be removed. In some embodiments, the film is water soluble and may be washed off while the indicia remains on the marijuana bud. One or more indicia may be applied on the film and then transferred to the marijuana bud.

Because the buds are eventually consumed by the end user, the marking material must be made from substances which are safe for ingestion, and/or which do not detrimentally alter the composition of the marijuana bud. For example, the marking material will typically comprise a liquid composition containing a liquid base and a colored substance. The liquid base can be aqueous or oil-based. An example of a suitable oil base is hemp oil. The liquid marking composition base may also include one or more active ingredients derived from the marijuana plant, including THC and/or CBD.

The colored substance contained in the liquid marking composition may comprise one or more approved food colorings, which may also have GRAS ("Generally Recognized As Safe") status.

In some embodiments, the marking material is configured to be invisible to the naked eye in normal transportation and usage conditions, for example, under sunlight or household lighting, while being visible in certain conditions, such as under a UV lamp. In some embodiments, the marking material becomes visible upon heating the marijuana bud to a predetermined temperature. For example, the marking material may become oxidized when heated, resulting in a color change such that the indicia become visible. For example, such materials include Cola drink, honey and sugar solution, lemon, apple, orange, onion juice, milk, soap water (the carboxylate in which partially oxidizes), wine, vinegar, and cobalt chloride. In some embodiments, the marking material becomes visible under UV light. For example, such materials include lemon juice. In some embodiments, the marking material becomes visible upon chemical reaction with another material such as an acid or a base. For example, the marking material may be vinegar, which becomes visible upon contact with red cabbage water. The marking material may be starch or lemon juice, which becomes visible upon contact with iodine solution.

The indicia may comprise various information. For example, the indicia may indicate the source of the marijuana bud, for example, the producer's name and/or logo.

It is well known that there are many varieties of marijuana with different characteristics. As such, the indicia may also indicate the particular variety of the marijuana.

The indicia may comprise information about the composition of the primary chemicals in the marijuana bud based on the variety. The indicia may further include information about the preferred method(s) to extract certain chemical from the marijuana bud. It is well known in the field that different marijuana varieties contain different chemicals or different amounts of the same chemical, for example, THC and CBD. As such, if a processor does not know the composition of at least the primary chemicals in the marijuana bud of the particular variety, the processor may not be able to effectively and efficiently extract the desired chemical. Worse still, the processor may use the wrong process to extract chemicals such that impurities or harmful chemicals may be extracted in a form that is not safe for consumption. For example, the extracted concentration may be too high, or the incorrect process may transform some harmless chemicals to harmful ones.

In addition, marijuana of different varieties may have different effects on the users. Without the relevant information, the user may inadvertently use the variety that is not suitable and/or safe for the user. Furthermore, to ensure safe use and to achieve the desired effects, the particular varieties of the marijuana may require specific method of consumption. Without the related information, the user may consume the marijuana bud in a way that does not achieve the desired effect or even in a way that is unsafe for the user.

By providing the information in the indicia that is applied to the marijuana bud, the marijuana processor and user can simply follow the information to ensure safe and satisfactory use of the marijuana bud. Without such indicia, it would be very inconvenient, if not impossible, to ensure safe and satisfactory use of the marijuana bud.

While various embodiments have been described in connection with the present disclosure, it will be understood that certain adaptations and modifications of the described exemplary embodiments can be made as construed within the scope of the present disclosure. Therefore, the above discussed embodiments are considered to be illustrative and not restrictive.

## Claims

1. A method for applying distinguishing markings to a marijuana bud, comprising:
**characterized by** applying one or more indicia directly to an irregular outer surface of the marijuana bud; and
in that the one or more indicia are applied to the marijuana bud by spraying, stamping or dipping.

2. The method according to claim 1, **characterized in that** the one or more indicia are applied by spraying the marijuana bud with a liquid marking composition comprising a liquid base and a colored substance.

3. The method according to claim 2, **characterized in that** the liquid base is aqueous or oil-based.

4. The method according to claim 2 or 3, **characterized in that** the liquid marking composition further comprises one or more active ingredients derived from the marijuana plant; and
**in that** the active ingredients are selected from one or more of THC (tetrahydrocannabinol) and CBD (cannabidiol).

5. The method according to any one of claims 2 to 4, **characterized in that** the colored substance comprises one or more food colorings.

6. The method according to any one of claims 1 to 5, **characterized in that** the method further comprises:
- moving the marijuana bud along a conveyor with a plurality of other buds;
- providing a plurality of spray nozzles above the conveyor;
- spraying the liquid marking composition directly onto the irregular outer surface of the marijuana bud from at least one of the plurality of spray nozzles.

7. The method according to claim 6, **characterized in that** at least one divider is disposed on the conveyor to form at least one channel such that movement of the marijuana bud is restricted in at least one of a direction of conveyor movement, and a lateral direction which is transverse to the direction of conveyor movement.

8. The method according to claim 1, **characterized in that** each of the indicia is stamped onto the outer surface of the marijuana bud by a stamping device.

9. The method of claim 8, **characterized in that** the stamping device has a flexible stamping surface.

10. The method according to any one of claims 1 to 9, **characterized in that** the indicia is invisible when applied; and
**in that** the indicia is visible when heated to a predetermined temperature, under UV light, or upon chemical reaction with another material.

11. An apparatus for applying indicia to a plurality of marijuana buds, comprising:
a conveyor defining a moving direction and a lateral direction which is transverse to the moving direction;
**characterized in that** the apparatus comprises:
a plurality of laterally-extending dividers spaced apart along the moving direction;
**in that** the dividers define a plurality of channels, each of the channels having a width sufficient to closely receive a marijuana bud; and
**in that** the width of each of the channels is defined along the moving direction.

12. The apparatus according to claim 11, **characterized in that** the channels have different widths, such that two or more marijuana buds of similar size may be received in the same channel.

13. The apparatus according to claim 11 or 12, **characterized in that** the apparatus further comprises a plurality of spray nozzles adapted to spray the indicia onto the outer surfaces of the marijuana buds received in the channels.

14. The apparatus according to claim 13, **characterized in that** the nozzles are movable along one or both of the moving direction and the lateral direction.

15. The apparatus according to claim 11 or 12, **characterized in that** the apparatus further comprises one or more stamping devices adapted to stamp the indicia onto the outer surfaces of the marijuana buds received in the channels; and
**in that** each of the stamping devices comprises a flexible stamping surface.
